# EUROPEAN PATENT APPLICATION

(11) **EP 1 862 462 A1**
(43) Date of publication of application: **05.12.2007**
(21) Application number: 07007368.9
(22) Date of filing: 10.04.2007
(51) Int. Cl.: C07D 401/04, A61K 31/4545, A61P 37/08

(54) **Process for the preparation of desloratadine**

(30) Priority: 10.04.2006 IN de09762006
(71) Applicant: Ranbaxy Laboratories Limited, Gurgaon - 122001, Haryana (IN)
(72) Inventor: Murpani, Deepak, New Delhi Delhi, 100024 (IN); Rafeeq, Mohammad, Pilibhit Uttar Pradesh 262121 (IN); Raheja, Praveen, New Delhi Delhi 100058 (IN); Sathyanarayana, Swargam, Karim Nager Andhra Pradesh 505002 (IN); Prasad, Mohan, Gurgaon Haryana 122001 (IN)
(74) Representative: Cronin, Brian Harold John

(57) **Abstract**

The present invention relates to an improved process for the preparation of Form 1 of desloratadine essentially free of Form 2. The invention also relates to Form 1 of desloratadine substantially free of Form 2, process for its preparation, pharmaceutical composition comprising it and its method of use for the treatment of allergic reactions in mammals.

## Description

### Field of the Invention

The present invention relates to an improved process for the preparation of Form 1 of desloratadine essentially free of Form 2, It also relates to Form 1 of desloratadine substantially free of Form 2, process for its preparation, pharmaceutical composition comprising it and its method of use for the treatment of allergic reactions in mammals.

### Background of the Invention

Desloratadine, which is chemically 8-Chloro-6, 11-dihydro-11-(4-piperidinylidene)-5H-benzo[5,6] cyclohepta [1,2-b] pyridine, is represented by Formula 1

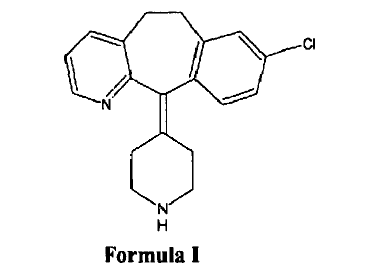

It is a metabolite of loratadine, having non-sedative antihistaminic activity and is known from U.S. Patent No. 4,659,716. Several methods for the preparation of desloratadine are known in the literature such as those described in U.S. Publication No. 2004242619, U.S. Patent No. 4,873,335, WO 2004/029039, which are herein incorporated by reference.

Desloratadine is known to exist in polymorphic Form 1, polymorphic Form 2 and amorphous form. Compositions comprising mixtures of crystalline forms 1 and 2 of desloratadine in specific weight to weight ratio are also known in the literature.

U.S. Patent No. 6,506,767 describes that desloratadine can exist in two distinct crystalline polymorphs, polymorph Form 1 of desloratadine essentially free of polymorph Form 2 and polymorph Form 2 of desloratadine substantially free of polymorph Form 1, each having distinctly different physical properties. The polymorph Form 1 of desloratadine essentially free of Form 2 is described as desloratadine having less than about 1% of Form 2.

WO 2004/080461 describes pharmaceutical compositions comprising stable mixtures of crystalline Forms 1 and 2 of desloratadine in specific weight to weight ratios and a pharmaceutically acceptable carrier.

A scientific update on desloratadine published by European Medicine Agency in 2004 mentions that no crystalline desloratadine was detected in the drug product using X-ray analysis when it is formulated by binding on a cation-exchange resin.

WO 2005/084674 describes amorphous form of desloratadine and process for its preparation.

WO 98/34614 describes pharmaceutical composition comprising large particles of desloratadine and a pharmaceutically acceptable carrier. The pharmaceutical composition has a particle size distribution in which greater than about 40% by weight of the desloratadine comprises particles having a size of 250 µm or larger.

The present invention provides an improved process for the preparation of Form 1 of desloratadine essentially free of form 2.

The invention also provides Form 1 of desloratadine substantially free of Form 2, processes for its preparation, pharmaceutical composition comprising it and its method of use for the treatment of allergic reactions in mammals.

### Summary of the Invention

A first aspect of the present invention provides an improved process for the preparation of Form 1 of desloratadine essentially free of Form 2 comprising the hydrolysis of loratadine and
a) extracting the reaction mixture with a first solvent;
b) partially recovering the first solvent;
c) adding a second solvent to the residue; and
d) isolating desloratadine Form 1 essentially free of Form 2.

A second aspect of the present invention provides Form 1 of desloratadine substantially free of Form 2.

A third aspect of the present invention provides a process for the preparation of Form 1 of desloratadine substantially free of Form 2 comprising the hydrolysis of loratadine and
a) extracting the reaction mixture with a first solvent;
b) partially recovering the first solvent;
c) adding a second solvent to the residue;
d) isolating desloratadine; and
e) compacting the desloratadine obtained in step (d).

A fourth aspect of the present invention provides a pharmaceutical composition comprising Form 1 of desloratadine substantially free of Form 2 and one or more pharmaceutically acceptable carriers, diluents or excipients and optionally other therapeutic ingredients.

A fifth aspect of the present invention provides methods of treating allergic reactions in mammals comprising administration of an anti-histamminic effective amount of Form 1 of desloratadine substantially free of Form 2.

"Form 1 of desloratadine essentially free of Form 2" herein refers to desloratadine Form 1 having less than 1 % of Form 2.

"Form 1 of desloratadine substantially free of Form 2" herein refers to desloratadine Form 1 having 1-15% of Form 2.

### Brief Description of the Drawings

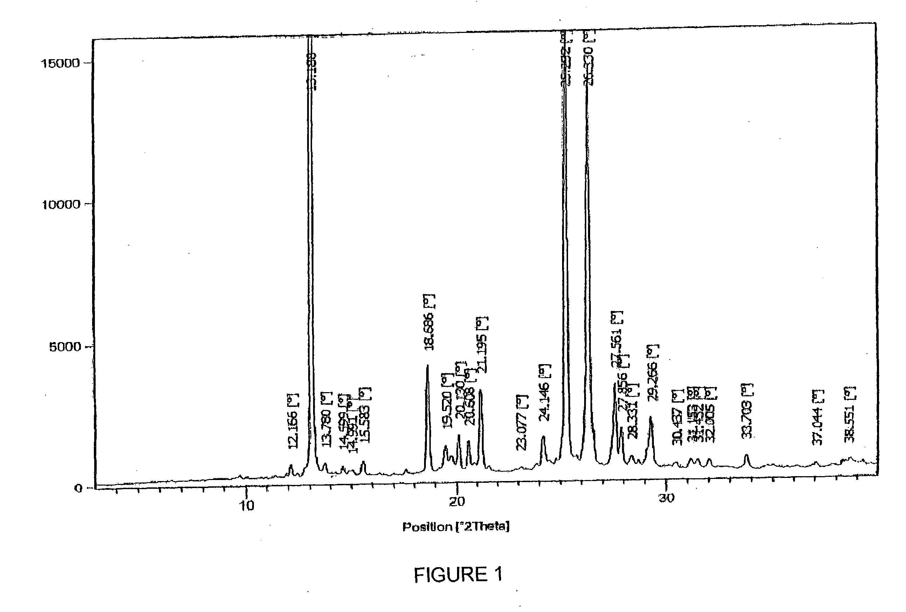

Figure 1 depicts the powder X-ray diffraction pattern of Form 1 of desloratadine essentially free of Form 2.

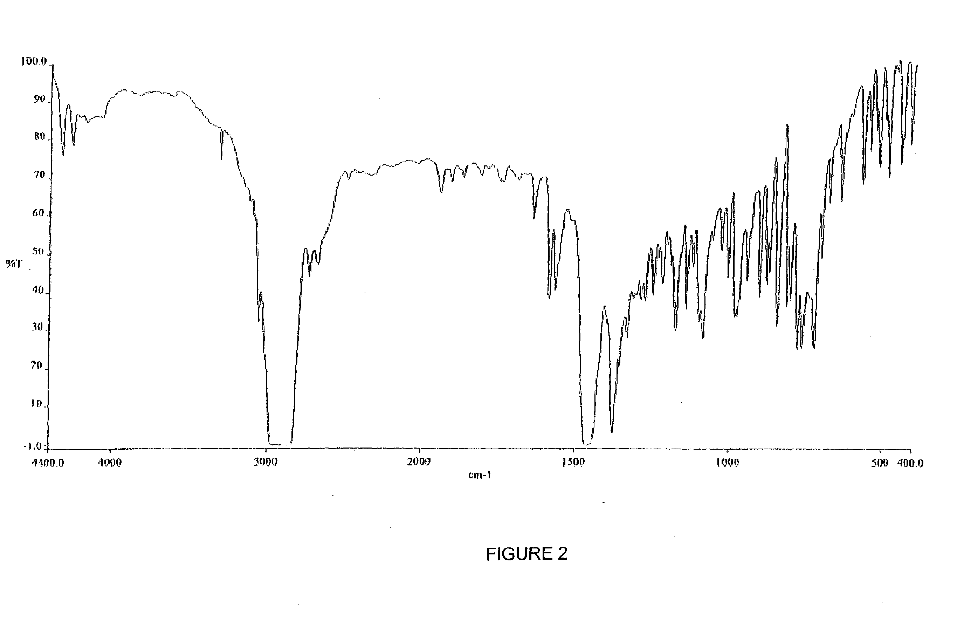

Figure 2 depicts the IR spectrum of Form 1 of desloratadine essentially free of Form 2.

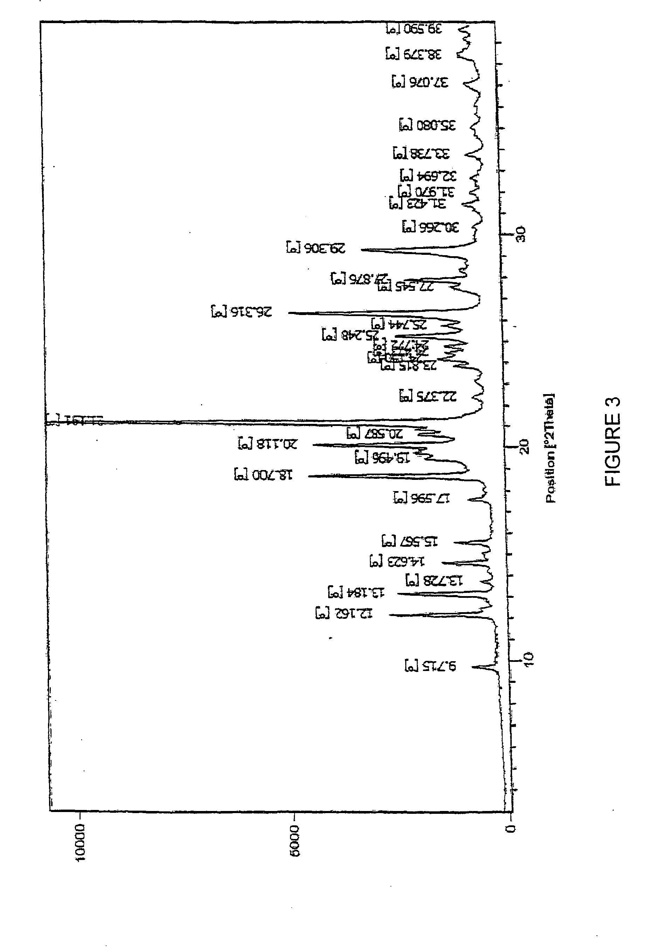

Figure 3 depicts the powder X-ray diffraction pattern of Form 1 of desloratadine substantially free of Form 2.

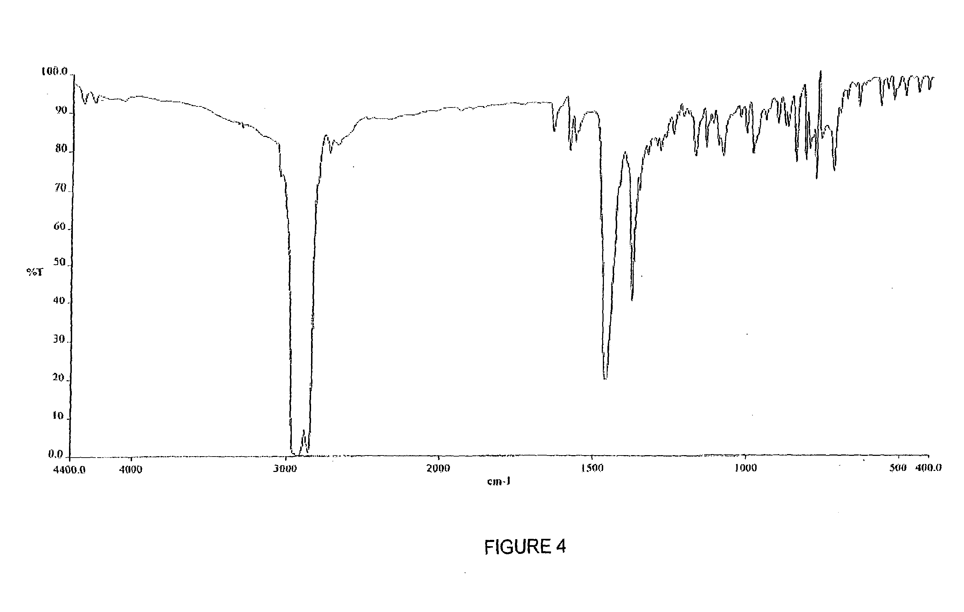

Figure 4 depicts the IR spectra of Form 1 of desloratadine substantially free of Form 2.

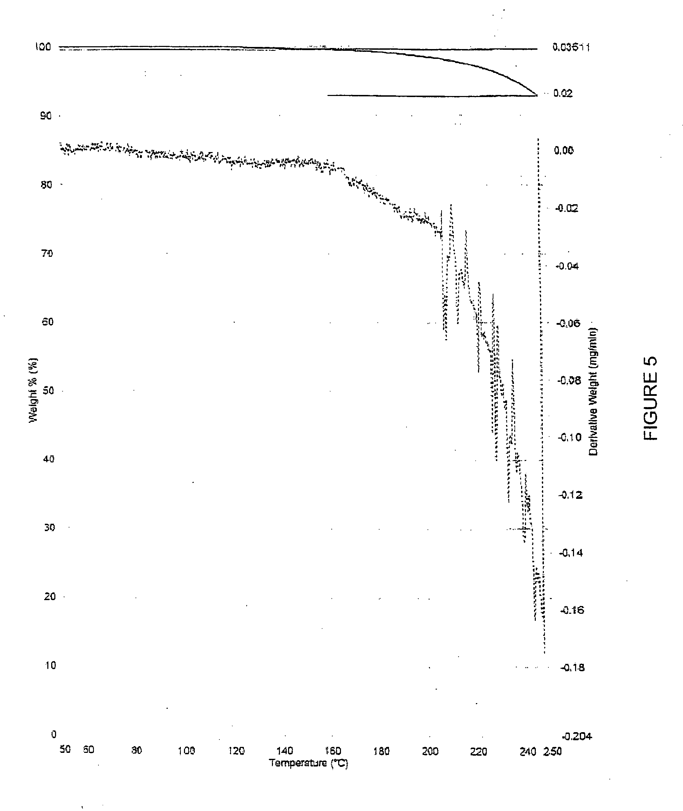

Figure 5 depicts the TGA of Form 1 of desloratadine substantially free of Form 2.

### Detailed Description of the Invention

Loratadine, to be used for the preparation of desloratadine, can be obtained by any of the processes known in the literature, such as those described in U.S. Patent Nos. 4,282,233, 6,271,378,6,084,100, and WO 2004/080997, which are herein incorporated by reference. The loratadine used as starting material may be obtained as a solution directly from a reaction mixture in which loratadine is formed and can be used as such without isolation.

Loratadine may be hydrolysed by conventional methods or by the methods known in the literature. In general, loratadine may be hydrolyzed in the presence of a base in a suitable solvent under reflux for 15-60 minutes. The base to be used for the hydrolysis of loratadine may be selected from alkali metal hydroxides. Preferably, the base used for hydrolysis is potassium hydroxide. The hydrolysis may be carried out in the presence of an inert gas.

The suitable solvent(s) for hydrolysis may be alcohols selected from the group comprising of straight and branched chain alcohols, cyclic alcohols, aromatic alcohols and/ or mixtures thereof. Examples of straight and branched chain alcohols may include methanol, ethanol, n-propanol, iso-propanol and/ or mixtures thereof. Examples of cyclic alcohols may include cyclopentanol, cyclohexanol and/ or mixtures thereof. Examples of aromatic alcohols may include substituted or un-substituted benzyl alcohols. The suitable solvent(s) to be used for hydrolysis may optionally contain water.

The first solvent to be used for the extraction of desloratadine may be selected from the group comprising of alkyl acetates, ethers, acetonilrile, N,N-dimethylformamide, chlorinated hydrocarbons, alcohols and/ or mixtures thereof. Examples of alkyl acetates may include ethyl acetate, iso-propyl acetate and/ or mixtures thereof. Preferably, the first solvent is ethyl acetate.

The reaction mixture may be washed with 10% aqueous sodium chloride solution. It may be further treated with a decolorizing agent such as activated carbon and filtered.

The first solvent may be partially recovered from the reaction mixture at a temperature of 60-85°C.

A second solvent may be added to the residue. The second solvent may be selected from the group comprising of ketones, alcohols, chlorinated hydrocarbons, ethers, acetonitrile, N,N-dimethylformamide, acetonitrile and/ or mixtures thereof. Examples of ketones may include acetone, methyl ethyl ketone, methyl n-propyl ketone and/ or mixtures thereof. Preferably, the second solvent is acetone.

The contents may be stirred for 1-5 hours. The reaction mixture may be cooled to 0-10°C, filtered and washed with a pre-cooled mixture of acetone and ethyl acetate and dried to obtain Form 1 of desloratadine essentially free of Form 2.

"Form 1 of desloratadine essentially free of form 2" herein refers to desloratadine Form 1 having less than 1% of Form 2.

Isolation may be accomplished by concentration, precipitation, cooling, filteration or centrifugation or a combination thereof followed by drying.

It has been observed that the amount of Form 2 of desloratadine in Form 1 of desloratadine having less than 1% of Form 2 can be increased by compaction to obtain Form 1 of desloratadine substantially free of Form 2. "Form 1 of desloratadine substantially free of Form 2" as used herein refers to Form 1 of desloratadine having 1-15% of Form 2.

Form 1 of desloratadine substantially free of Form 2 may be obtained by carrying out the steps (a-d) in a manner similar to that described in the first aspect of the present invention followed by compaction.

The compaction may be carried out using a roller compactor at ambient temperature and at a hydraulic pressure of 40-80 bar to obtain Form 1 of desloratadine substantially free of Form 2.

A roller compactor generally incorporates two or more rollers adjacent and parallel to each-other with a fixed or adjustable gap between the two rollers. A hopper or other feeding device deposits the material to be compacted between the moving rollers. Roller compactors are typically equipped with dividers that cut or otherwise divide the compacted material emerging from the roller compactor into ribbons.

The product obtained after compaction is then broken apart to form granules, typically by suitable mechanical means such as by crushing, grinding or cutting. For example, granules may be formed from the compact by pulverization.

Pulverization involves subjecting the granules to shear force such that the desired particle size of the granulation is achieved. Pulverization can be carried out using various types of mills such as air-jet mill, pin mill and hammer mill. The milled material may be optionally screened. In screening, the material is passed through a mesh screen or series of mesh screens to obtain the desired particle size. The sieved material can be micronized using a fluid energy mill for 10-40 minutes.

Desloratadine Form 1 substantially free of Form 2 obtained by the process of the present invention has constant physicochemical properties (in terms of dissolution, content uniformity, bioavailablity and the like) and is compatible with a wide range of reactive excipients in compliance with GMP requirements. The reactive excipients may include lactose, starch, mono- and disaccharides and the like.

Desloratadine Form 1 substantially free of Form 2 may be characterized by its powder X-ray diffraction pattern, differential scanning calorimetry and infra-red spectrum.

Form 1 of desloratadine substantially free of Form 2 may be characterized by a powder X-ray diffraction pattern with strong peaks at about 18.70, 20.11, 21.19, 26.31 and 29.30 ± 0,2 degrees two-theta and weak peaks at about 12.16, 13.18, 20.58, 25.24 and 27.87, ± 0.2 degrees two-theta.

Desloratadine Form 1 substantially free of Form 2 of the present invention may comprise small particles wherein the size of 90% of the particles is not more than 40 µm.

Desloratadine Form 1 substantially free of Form 2 obtained by the process of the present invention possesses anti-histamminic properties with no sedative effects. It is usually administered as a part of a pharmaceutical composition. Pharmaceutical compositions of this invention may contain in addition to an anti-histamminic effective amount of desloratadine Form 1 substantially free of Form 2, inert pharmaceutically acceptable carriers that may be solid or liquid.

Solid form compositions include powders, tablets, dispersible granules, capsules, cachets, and suppositories. Liquid form preparations include solutions, suspensions and emulsions. The compositions comprise of small particles having a size of not more than 40 µm.

Topical formulations useful for nasal or ophthalmic administration are also contemplated. Topical formulation suitable for nasal administration may be solutions or suspensions. Ophthalmic formulations may be solutions, suspension or ointments. Ointments usually contain lipophilic carriers such as mineral oil and/or petrolatum. Solution for ophthalmic administration may contain sodium chloride, acid and/or base to adjust the pH as well as purified water and preservatives.

In the foregoing section embodiments are described by way of example to illustrate the process of invention. However, this is not intended in any way to limit the scope of the present invention. Several variants of the example would be evident to persons ordinarily skilled in the art.

### Methods

Powder XRD
   X-Ray Difractometer, Rigaku Coorperation, RU-H3R
   Goniometer CN2155A3
   X-Ray tube with Cu target anode
   Divergence slits 1 0, Receiving slit 0.15mm, Scatter slit 1 0
   Power: 40 KV, 100 mA
   Scanning speed: 2 deg/min step: 0.02 deg
   Wave length: 1.5406 A
IR
   Perkin Elmer FT-IR spectrophotometer
DSC
   Mettler Toledo instrument
Roll Compactor
   Cadmach
Multimill
   Magumps
Air Jet Mill
   Hosokawa Alpine Aktiengesellschaft & Co. OHG

### Example 1:

To a solution of potassium hydroxide (120 g) in ethanol (694 ml) and water (70 ml) was added loratadine (100 g) at 43-45°C under an atmosphere of nitrogen. The contents were refluxed for 12-14 hours. The reaction mixture was monitored by HPLC and was continued till un-reacted loratadine was not more than 1%. The reaction mixture was cooled to 35-40°C and de-ionized water (625 ml) was added. Ethanol was recovered under reduced pressure at a temperature of 50-55°C. The residue was extracted twice with ethyl acetate (500 ml+300 ml) at room temperature. The organic layer was washed with 10% sodium chloride solution, decolorized with activated carbon, dried with sodium sulphate and filtered through hyflo bed. The organic layers were combined and ethyl acetate was recovered at a temperature of 76-78°C at atmospheric pressure leaving inside a total volume of about 160 ml. The contents were cooled to 20-25°C. Acetone (200 ml) was then added to the reaction mixture and it was stirred for 3 hours. The reaction mixture was then cooled to 0-5°C, filtered, washed with pre-cooled mixture of acetone and ethyl acetate and dried under reduced pressure at 50-55°C to obtain Form 1 of desloratadine essentially free of Form 2.
**Yield:** 68 g
**HPLC Purity:** 99.5%

### Example 2

Desloratadine (67 g) obtained in example 1 was subjected to compaction using a screw feeder having 35 revolutions per minute and a roll compactor having 03 revolutions per minute at ambient temperature and at a hydraulic pressure of 60 bar to obtain Form 1 of desloratadine substantially free of Form 2.

### Example 3

Desloratadine obtained in example 2 above was pulverized for 10-15 minutes, sieved through 60 mesh size screen to obtain Form 1 of desloratadine substantially free of Form 2.

### Example 4

Desloratadine obtained in example 3 above was micronized for 20-25 minutes to obtain Form 1 of desloratadine substantially free of Form 2.
Yield: 60 g

## Claims

1. An improved process for the preparation of Form 1 of desloratadine essentially free of Form 2 comprising the hydrolysis of loratadine and
a) extracting the reaction mixture with a first solvent;
b) partially recovering the first solvent;
c) adding a second solvent to the residue; and
d) isolating desloratadine Form 1 essentially free of Form 2.

2. Form 1 of desloratadine substantially free of Form 2.

3. Desloratadine according to claim 2 having X-ay powder diffraction pattern as depicted in Figure 3.

4. Desloratadine according to claim 2 having the DSC thermogram as depicted in Figure 4.

5. Desloratadine according to claim 2 having the Infra-red spectrum as depicted in Figure 5.

6. Desloratadine according to claim 2 comprising small particles wherein the size of 90% of particles have a size of not more than 40 micron.

7. A process for the preparation desloratadine according to claim 2 comprising the hydrolysis of loratadine and
a) extracting the reaction mixture with a first solvent;
b) partially recovering the first solvent;
c) adding a second solvent to the residue;
d) isolating desloratadine; and
e) compacting the desloratadine obtained in step (d).

8. The process according to claims 1 or 7, wherein the first solvent is selected from the group comprising of alkyl acetates, ethers, acetonilrile, N, N-dimethylformamide, chlorinated hydrocarbons, alcohols and/ or mixtures thereof.

9. The process according to claims 1 or 7, wherein the second solvent is selected from the group comprising of ketones, alcohols, chlorinated hydrocarbons, ethers, acetonitrile, N,N-dimethylformamide, acetonitrile and mixtures thereof.

10. The process according to claim 7, wherein the compaction is carried out using a roller compactor at a hydraulic pressure of 40-80 bar.

11. The process according to claim 7, wherein the compact material is pulverized and then micronized.

12. A pharmaceutical composition comprising Form 1 of desloratadine substantially free of Form 2 and one or more pharmaceutically acceptable carriers, diluents or excipients and optionally other therapeutic ingredients.

13. The pharmaceutical composition of claim 12 wherein the size of 90% of the particles of desloratadine is not more than 40 micron.

14. A method of treating allergic reactions in mammals comprising administration of an anti-histamminic effective amount of Form 1 of desloratadine substantially free of Form 2.
